(19) Europäisches Patentamt / European Patent Office / Office européen des brevets

(11) **EP 4 427 233 B1**

(12) **EUROPEAN PATENT SPECIFICATION**

(45) Date of publication and mention
of the grant of the patent:
**09.04.2025 Bulletin 2025/15**

(21) Application number: **22813974.7**

(22) Date of filing: **04.11.2022**

(51) International Patent Classification (IPC):
**G16H 20/10** *(2018.01)* **G16H 50/50** *(2018.01)*

(52) Cooperative Patent Classification (CPC):
**G16H 50/50; G16H 20/10**

(86) International application number:
**PCT/EP2022/080865**

(87) International publication number:
**WO 2023/079106 (11.05.2023 Gazette 2023/19)**

(54) **METHOD, SYSTEM AND COMPUTER PROGRAM FOR COMPUTATION OF OPTIMAL INDIVIDUAL DOSING REGIMEN, PARTICULARLY SUBJECT TO CLINICAL CONSTRAINTS**

VERFAHREN, SYSTEM UND COMPUTERPROGRAMM ZUR BERECHNUNG VON OPTIMALEN INDIVIDUELLEN DOSIERUNGSSCHEMAS, INSBESONDERE UNTER KLINISCHEN EINSCHRÄNKUNGEN

PROCÉDÉ, SYSTÈME ET PROGRAMME INFORMATIQUE POUR LE CALCUL D'UN RÉGIME DE DOSAGE INDIVIDUEL OPTIMAL, PARTICULIÈREMENT SOUMIS À DES CONTRAINTES CLINIQUES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **04.11.2021 EP 21206570**

(43) Date of publication of application:
**11.09.2024 Bulletin 2024/37**

(73) Proprietors:
• **Universität Basel**
 **4001 Basel (CH)**
• **Universität Konstanz**
 **78464 Konstanz (DE)**

(72) Inventors:
• **SZINNAI, Gabor**
 **4102 Binningen (CH)**
• **KOCH, Gilbert**
 **88662 Überlingen (DE)**
• **PFISTER, Marc**
 **3652 Hilterfingen (CH)**
• **SCHROPP, Johannes**
 **78462 Konstanz (DE)**
• **STEFFENS, Britta**
 **78464 Konstanz (DE)**
• **BACHMANN, Freya**
 **78467 Konstanz (DE)**

(74) Representative: **Bittner, Peter et al**
**Peter Bittner und Partner**
**Herrenwiesenweg 2**
**69207 Sandhausen (DE)**

(56) References cited:
**US-A1- 2020 273 578 US-A1- 2020 350 073**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a method, a system and a computer program for determining an optimal dosing regimen of at least one drug for a patient suffering from a known disease.

A majority of diseases are still difficult to treat at the individual level as disease progression differs among patients and clinical practice. Particularly, it often remains a challenge to determine the optimal individual dosing regimen, particularly in a clinical setting, in a sufficiently fast and reliable fashion that ideally allows to control the disease progression, while minimizing treatment related adverse events, resulting in recovery of the patient.

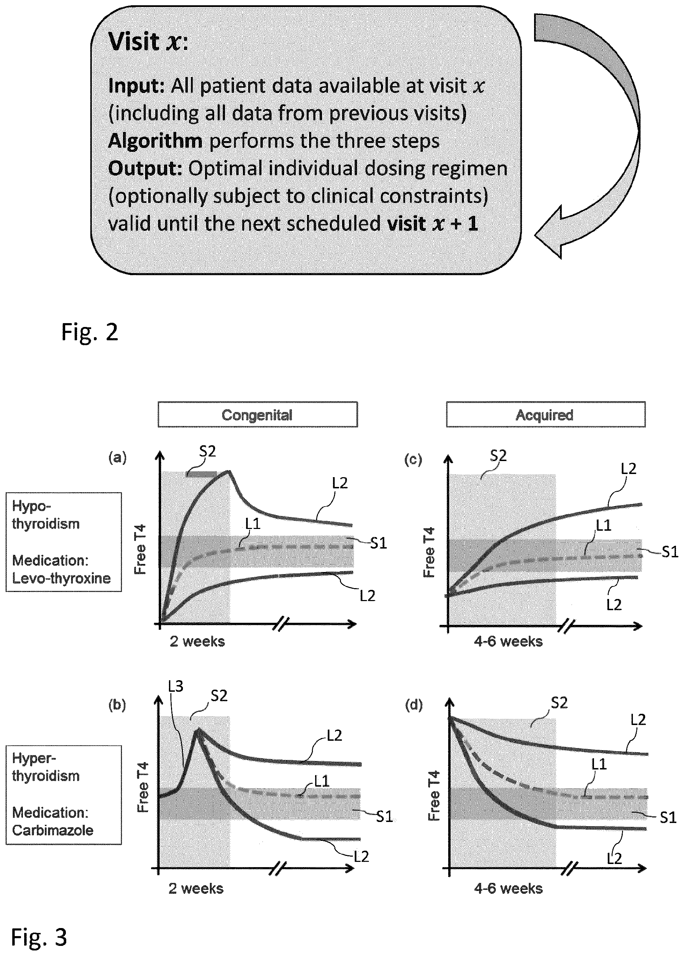

Particularly, among such diseases, congenital hypothyroidism is the most frequent congenital endocrine disorder and the most frequent preventable cause of mental retardation worldwide [Polak 2017]. Disease severity strongly varies among individuals at diagnosis, i.e. the thyroid-stimulating hormone (TSH) is in the range of 6-1200mU/L whereas the norm is 0.5-5 mU/L. Main effects of thyroid hormones are axonal outgrowth of interneurons and myelination, processes that are ongoing in humans mainly after birth. Neonatal screening allows diagnosing of children with congenital hypothyroidism, before they suffer from clinical signs and irreversible neurological deficiencies. During the last 40 years, the neurological outcome of patients with congenital hypothyroidism has been strongly improved 1) by shortening the time window between screening and onset of treatment (currently 4-5 days in Switzerland) and 2) by increasing starting doses of levothyroxine (from 5-8 mcg/kg/d to currently 10-15 mcg/kg/d). The goal of substitutive treatment with levothyroxine is to correct hypothyroidism as rapidly as possible to protect brain development. The clinician aims to reach free thyroxine (T4) levels in the upper normal reference range within 14 days (cf. Fig. 3 (a)). However, several studies revealed that up to 10% of children were overdosed with a starting and maintenance dose of 10-15 mcg/kg/d over relevant time periods. Further, at age of 11 years their neurological outcome was comparable to children with periods of underdosing during follow-up, and both over- and underdosed children had a worse neurological outcome compared to children with levothyroxine doses maintaining thyroid hormones in the reference range during follow-up.

Current international guidelines recommend more frequent controls during the first 2 years of life, to allow more frequent and necessary individual, age-appropriate dose adjustments maintaining thyroid hormones in a physiological normal reference range. Although this permits a faster detection of over- and underdosing, it does not prevent episodes of suboptimal dosing. As a consequence, the last step for improving neurological outcome in affected patients must focus on optimization of long-term substitutive doses by considering clinical and laboratory data for personalized dosing during follow-up. Therefore, it is particularly highly desirable to be able to determine optimal personalized doses during the first 2 years of life, which are essential for brain development and long-term normal cognitive function. Central congenital hypothyroidism is a subgroup of congenital hypothyroidism with low TSH and FT4 values due to disorders of the pituitary gland and the hypothalamus treated with levothyroxine in the same way.

Furthermore, congenital hyperthyroidism occurs in about 10% of offspring of mothers with autoimmune hyperthyroidism (Graves' disease) due to transplacental passage of stimulating antibodies against the thyrotropin (TSH)-receptor. It puts infants to significant risk for morbidity and mortality. In contrast to infants with congenital hypothyroidism, who are in 95% asymptomatic at birth and during the first weeks of life, infants suffering from congenital hyperthyroidism present with typical signs and symptoms, ranging from mild tachycardia and sweating to weight loss, vomiting, diarrhoea, dehydration, severe hyperthermia, seizures, and cardiac insufficiency. Onset is either directly at birth or delayed by transplacental passage of maternal anti-thyroid medication until day 4-10 of life. Again, severity of disease is very variable ranging from very mild forms to life-threatening complications. The delayed onset further complicates optimal treatment, making clinical observation and laboratory controls mandatory at day 1, 4, 7, 10 and 14 of life.

Also, for congenital hyperthyroidism optimizing the treatment by anticipating which child is at risk for severe clinical course and when treatment should be started, and by finding the minimal effective starting and maintenance dose adapted for disease severity in the context of possible severe side effects of anti-thyroid drugs (agranulocytosis, hepatitis, vasculitis) is highly desirable (cf. Fig. 3 (b)).

Furthermore, acquired hyperthyroidism [Leger 2014] occurs in 0.5-2:100000 in children in Europe and in 25-90:100000 in adults in Europe, and has highest prevalence in Brazil, China and India ranging from 0.75-1.25% of the population, respectively. Severity of hyperthyroidism again strongly varies among individuals at diagnosis, with FT4 values ranging from 22 to >100 pmol/L whereas the norm is 11-21 pmol/L. Restoration of physiological FT4 levels should be achieved after 4-6 weeks, in contrast to congenital thyroid disease, where normalization should be aimed as rapidly as possible. A simple treatment approach was in former years a block-and-replace strategy: The dose of anti-thyroid drugs was chosen to completely block the thyroid, and substitution with levothyroxine was added. According to international guidelines, in the context of increasing frequency of side effects with higher doses of anti-thyroid drugs (agranulocytosis, hepatitis, vasculitis), such an approach is not recommended anymore according to international guidelines. Currently, the minimal effective dose of the anti-thyroid agent has to be titrated on an individual level to mitigate the risk of overdosing causing iatrogenic hypothyroidism or underdosing resulting in persistent hyperthyroidism. Both hyper- and hypothyroidism have negative effects on growth and pubertal development (retardation or acceleration, respectively), and on cognitive function leading to a decrease in school and sport performance. Finally, to further increase complexity during childhood, the disease course is more severe and risk of hyperthyroidism recurrence is much higher in young or prepubertal children compared to older (>10 years) or pubertal and postpubertal children, rendering childhood Graves' disease a therapeutic challenge.

Also for Graves' disease, treatment strategies that allow one to compute the optimal individual starting dose to reach steady-state and minimal effective maintenance dose to avoid recurrence during follow-up depending on clinical and laboratory parameters are highly desirable (cf. Fig. 3 (d)).

Furthermore, hypothyroidism is common throughout the world in an adult population with a prevalence ranging from 0.2-5.3% in Europe. The most frequent cause of acquired hypothyroidism is Hashimoto thyroiditis in iodine sufficient areas of the world. The aim for treatment of acquired hypothyroidism (incidence rate of 1.2%) is to normalize free T4 levels slowly, to avoid signs and symptoms of hyperthyroidism after longstanding hypothyroidism. In analogy with the other diseases, an optimal starting and maintenance dose is necessary to normalize somatic growth, cognitive function and consequently school performance but not overdose the pediatric patient (cf. Fig. 3 (c)). Further, central hypothyroidism due to disorders of the pituitary gland and the hypothalamus (e.g. trauma, tumor, infection, a.o.) may cause a further form of acquired hypothyroidism.

Document US 2020/0273578 A1 discloses a method and a system to determine an optimal individual regimen for a patient suffering from a specific disease.

In summary, based on the above, the problem to be solved by the present invention is to provide a method, a system, a computer program, a computer-readable non-transitory storage medium, and a data carrier signal that allow to improve personalized dose finding, particularly for pediatric thyroid diseases. Due to the severity of such diseases it is of high clinical relevance to improve treatment and outcome of affected patients.

This problem is solved by a method having the features of claim 1, a system having the features of claim 10, a computer program having the features of claim 11 a computer-readable non-transitory storage medium having the features of claim 12 and by a data carrier signal having the features of claim 14.

Preferred embodiments of these aspects of the present invention are stated in the corresponding dependent claims and are described below.

Advantageously, the present invention allows a personalized dosing so as to raise medical therapy to a next level. As for many diseases no new pharmacologic approaches are under development, particularly not for hyper- or hypothyroidism, improving on determining individual dosing regimen is the key in improving therapy outcomes.

Particularly, as outlined above and indicated in Figure 3, the present invention is particularly applicable to modeling the different clinical situations that require to be modeled separately to develop disease specific algorithms for starting and maintenance dosages.

In the framework of the present invention, the following notions and definitions are frequently referred to.

Patient

A patient can be a virtual / in-silico (non-human) object (e.g., the average of a patient population) or a real human patient.

Visit

A visit of a patient can be (i) any time point of interest, (ii) the time point of a clinical visit of a non-hospitalized patient at the hospital, (iii) or the time point at which the clinician sees a hospitalized patient in the hospital.

Wearable devices and sensors

Wearable devices and sensors are noninvasive devices that are worn by the patient and monitor vital signs such as heart rate (beats per minute), blood pressure (mmHg), body temperature (°C), respiratory rate (frequency/minute), oxygen saturation and steps (counts per day).

Laboratory measurements

Laboratory measurements are routine biochemical tests of biomarkers (e.g., hormones) analyzed in medical laboratories or by health care providers like physicians, nurses, medical assistants or pharmacists. In case of thyroid hormones, one hormone is synthesized and secreted by the pituitary gland (regulatory hormone of the peripheral endocrine gland called thyrotropin or thyroid-stimulating-hormone (TSH) for the pituitary thyroid axis) and peripheral hormones are secreted from the thyroid (endocrine gland under control of the pituitary gland which are thyroxine (T4) and triiodothyronine (T3) in bound (T3, T4) and free forms (FT3, FT4)).

Demographic / anthropometric characteristics

Demographic data are, e.g., age (days (neonate), months (infant), years (child or adult)), gestational age at birth (premature or term born) and gender. Anthropometric data are, e.g., body weight (kg), height (cm) and head circumference (cm).

Covariates

A covariate is a patient characteristic (e.g., a demographic / anthropometric characteristic) that can, but not necessarily must, vary over time. A time-invariant covariate is e.g., birth weight, gestational age, delivery mode, all characteristics at baseline etc. Examples for a time-varying covariate are weight, height, heart rate, blood pressure, respiratory rate etc.

Patient data

The patient data is the combination of data from a wearable device or sensor, laboratory measurements, and covariates from all available visits. In addition, the patient data contains all information about administered doses from all available visits.

Pharmacokinetic model

The pharmacokinetic (PK) model describes the concentration of a drug (or e.g., its metabolite) or an exogenous substance, by e.g., absorption, distribution, metabolism and elimination processes. Route of administrations of the drug can be (i) oral, (ii) intravenous, or (iii) subcutaneous. A PK model is typically characterized by one or more differential equations.

Pharmacokinetic / Pharmacodynamic model

The pharmacokinetic / pharmacodynamic (PKPD) model describes the response of a biomarker or a disease progression etc. (the so-called pharmacodynamic) caused by stimulatory / inhibitory effects of one or several drugs e.g., characterized by a PK model. For a combination of several drugs, the drugs can be administered simultaneously or sequentially. A PKPD model is typically characterized by one or more differential equations.

Basic Mathematical model

The basic mathematical model can be a PK model only, or a PKPD model. The basic mathematical model may include a combination of physiologically based mechanistic assumptions, maturation related processes and/or data-driven assumptions. The basic mathematical model is controlled by the administered doses of one or several drugs. The basic mathematical model contains model parameters summarized in the vector $\theta$, some of them might be already known while others need to be estimated. The disease state is one state variable or a combination of several state variables of the basic mathematical model that characterizes the disease progression.

Individual model parameters

The individual model parameters are summarized in the vector $\theta_i$ and characterize an individual patient based on the individual data. Index i denotes the i-th individual in a population with $N$ patients, i.e., $i = 1, ..., N$. All or just some model parameters can have physiological, biological or other meanings. For example, a model parameter can describe the rate of a process (e.g., production or elimination), can describe the volume of a compartment, or can have other meanings.

Desired disease progression

The desired disease progression is given by the clinician / user and characterizes the goal for the individual patient. For example, for a patient showing an increased biomarker that is treated with a drug, the goal is to normalize the biomarker value to the normal (age-specific) reference range within a given time period. Or, in the example of a PK model, the desired disease progression can be a goal regarding drug-exposure, e.g., achieving a certain area under the concentration curve. The desired disease progression under pharmacological therapy is characterized by a mathematical function.

Dosing regimen

The dosing regimen is the information about the administration of one drug or several drugs. It contains the drug(s) itself, the route of administration (oral, intravenous, subcutaneous), the dosing time points, and the doses.

Optimal individual dosing regimen

The optimal individual dosing regimen is the dosing regimen with the optimal (with respect to the desired disease progression) doses for an individual patient.

Optimal individual dosing regimen subject to constraints in clinical application

The optimal individual dosing regimen subject to constraints in clinical application is the optimal individual dosing regimen adjusted to constraints in clinical application. This can be, e.g., available tablet sizes. Different strategies can be applied to adjust an optimal dose between two available dosing sizes, e.g.,

1) Round to the closest available dosing size, or
2) Choose upper or lower available dose based on the lower corresponding cost functional value (as in mixed-integer optimization [Belotti 2013])

Non-linear mixed effects modeling approach

In the non-linear mixed effects (NLME) modeling approach [Lavielle 2014] it is assumed that patients with the same disease form a population having common or similar features. For each individual $i$, the vector of $n_i$ measurements $w_i$ = $(w_{i1}, \dots, w_{in_i})$ at time points $t_i = (t_{i1}, \dots, t_{in_i})$ are taken for $i = 1, \dots, N$. The prediction $f$ from the basic mathematical model forecast these measurements. However, the individual model parameters $\theta_i$ in the basic mathematical model are unknown or at least partially unknown. A subset of these model parameters are the estimated individual model parameters $\phi_i$. The basic NLME modeling approach reads

$$\phi_i = \phi_{pop} + \beta c_i + \eta_i$$

$$w_{ij} = f\big(t_{ij}, \phi_i\big) + \epsilon_{ij}$$

for $i = 1, \dots, N$ and $j = 1, \dots, n_i$ where $\eta_i$ are the random effects with $\eta_i \sim \mathcal{N}(0, \Omega)$ and covariance matrix $\Omega$, $\beta$ denotes the covariate effects, $c_i$ the individual covariate values, and $\phi_{pop}$ the parameters of the typical (average) individual in the population. The individual model parameters $\theta_i$ utilized in the basic mathematical model contain the estimated individual model parameters $\phi_i$ as well as known parameters, and $\varepsilon_i$ is the residual error for which different error models can be considered. Hence, the individual model parameters $\phi_i$ are treated as random variables and are assumed to be independently and normally distributed. Other distributions can be realized by transformation $\phi = h(\psi)$, e.g., $h(x) = \log(x)$ for log-normally distributed model parameters $\psi$. The population parameters $\rho = (\phi_{pop}, \Omega, \beta)$ are estimated by maximizing the so-called observed likelihood

$$p((w_1, \dots, w_N); \rho) = \prod_{i=1}^{N} \int p(w_i | \phi_i; \rho)\, p(\phi_i; \rho)\, d\phi_i$$

**(equivalently** the log-likelihood) describing the probability to achieve the observed measurements $w_1, \dots, w_N$ with respect to $\rho$, i.e., the $\rho$ is chosen such that the model most likely predicts the observed measurements. The observed likelihood depends on the conditional probability density functions $\rho(w_i | \phi_i; \rho)$ and the marginal probability density functions $p(\phi_i; \rho)$.

Mathematical model

The mathematical model is a basic mathematical model together with the application of the NLME modeling approach to describe a given population of patients by its population parameters $\rho$. Such a mathematical model is therefore sometimes also denoted as fully developed mathematical model.

Empirical Bayesian Estimation

The empirical Bayesian estimation also known as maximum a posteriori estimation [Bassett, Deride 2019] computes the estimated individual model parameters $\phi_i$ which most likely predict the observed measurements $w_i$ at time points $t_i$ for the individual i belonging to a population with population parameters $\rho$. Mathematically, the conditional probability density function $p(\phi_i|w_i; \rho)$ is maximized or equivalently, minus twice the log-likelihood is minimized, i.e.,

$$\phi_i = \arg\min\left\{-2\log p(\phi_i|w_i;\rho)\right\}$$

Optimal Control Problem

An optimal control problem is solved to compute a control (i.e., the doses of one or several drugs) such that one state variable or a combination of several state variables of the mathematical model (e.g., the disease state of the patient) reaches the desired disease progression as closely as possible. This is achieved by minimizing a cost functional which characterizes the difference between the disease state resulting from a particular control and the desired disease progression. The optimal control problem can be solved with an optimal control algorithm such as the OptiDose algorithm disclosed in the article: "Bachmann F, Koch G, Pfister M, Szinnai G, Schropp J (2021) OptiDose: Computing the Individualized Optimal Drug Dosing Regimen Using Optimal Control. Journal of Optimization Theory and Applications 189:46-65", which is hereby incorporated herein by reference in its entirety. Particularly, the optimization is an iterative procedure (i) starting with an initial control, (ii) solving the mathematical model to obtain the disease state, (iii) solving the adjoint equation (linking disease state and cost functional) and (iv) updating the control utilizing the gradient of the cost functional. Steps (ii)-(iv) are repeated in each iteration.

According to a preferred embodiment of the method according to the present invention, the method further comprises the step of:

(d) Adjusting the optimal individual dosing regimen to account for at least one clinical constraint to yield the optimal individual dosing regimen subject to said at least one clinical constraint.

Further, according to an embodiment of the method, the mathematical model is or comprises a preferably pharmacometric, pharmacokinetic-pharmacodynamic (PKPD) model.

Further, according to an embodiment of the method, the desired disease progression is given in form of a mathematical function.

Further, according to an embodiment of the method, steps (b) to (c), particularly (b) to (d), are conducted at each visit of a plurality of $x$ succeeding visits of the patient, wherein for $x > 1$ the patient data in step (b) includes all patient data of the previous $x$ - 1 visit(s) and the empirical Bayesian estimation in step (b) further uses all doses of said at least one drug actually administered to the patient until the current visit.

Further, according to an embodiment of the method, at least a part of an algorithm performing steps (b) to (c), particularly (b) to (d), is approximated by an artificial neural network (ANN).

Artificial neural networks (ANN) are universal approximators and can therefore approximate any input/output relationship up to a certain accuracy depending on the amount of available training data and the structure of the ANN. Hence, an ANN can also approximate any algorithm up to a certain accuracy, i.e., it can learn the functionality of the algorithm and finally mimic the algorithm. The advantage of a trained ANN is that it can compute the learned input/output relationship in a split second on standard computers and hence can replace a computationally time-consuming algorithm.

Particularly, according to a preferred embodiment, the steps (a) to (c), and optionally step (d) are approximated by an ANN.

Particularly, for this, in an embodiment, multiple simulations with the respective part of the algorithm / step of the method that shall be substituted/approximated by the ANN are conducted, wherein these simulations are then used as training data for the ANN, and wherein resulting trained ANN will be included in the iterative algorithm (or used in the method) instead. Hence, the computational cost of some parts of the algorithm can be outsourced into the algorithm development by training an ANN upfront. This can significantly reduce the computational cost for daily clinical application.

Furthermore, according to an embodiment of the method, the disease is one of: acquired hypothyroidism (autoimmune and non-autoimmune forms), acquired hyperthyroidism (autoimmune and non-autoimmune forms), congenital hypothyroidism, congenital hyperthyroidism.

Furthermore, according to an embodiment of the method, the drug is selected from the group comprised of: levothyroxine ($C_{15}H_{11}I_4NO_4$, CAS number 51-48-9) for treatment of any form of hypothyroidism, carbimazole ($C_7H_{10}N_2O_2S$, CAS number 22232-54-8), methimazole ($C_4H_6N_2S$, CAS number 60-56-0), propylthiouracil ($C_7H_{10}N_2OS$, CAS number 51-52-5) for treatment of any form of hyperthyroidism.

Furthermore, according to an embodiment of the method, at least a portion of said patient data is measured by a wearable device worn by the patient.

Wherein the wearable device is one of: a watch, particularly a smart watch, a mobile phone, particularly a smart phone. Particularly, a smart phone is a mobile phone comprising at least one processor configured to execute computer programs and a display for displaying information, wherein the display can be a touch screen forming part of a user interface. Likewise, a smart watch can in particular be a watch comprising at least one processor configured to execute computer programs and a display for displaying information, wherein the display can be a touch screen forming part of a user interface of the watch.

Further, according to an embodiment of the method according to the present invention, said patient data comprises a vital sign such as the heart rate of the patient, wherein the vital sign (e.g. heart rate) of the patient is measured with the wearable device. Other vital signs/physiological data that can be measured by the wearable device are at least one of: blood pressure (mmHg), body temperature (°C), respiratory rate (frequency/minute), oxygen saturation, steps (counts per day).

Yet another aspect of the present invention relates to a system for automatically determining an optimal individual dosing regimen of at least one drug for a patient suffering from a known disease (the optimal individual dosing regimen being optionally subject to at least one clinical constraint), the system comprising at least one processor configured to perform the steps of the method according to the present invention.

According to an embodiment of the system, the system further comprises a wearable device configured to measure at least a portion of said patient data. Particularly, the wearable device can be configured to measure physiological data of the patient. In an embodiment the wearable device can be configured to measure a heart rate.

A computer program comprising instructions, which when carried out on a computer or on the system according to the present invention, cause the computer (or system) to carry out the steps of the method according to the present invention. Particularly, the system or computer is caused to receive patient data, particularly a heart rate measured by the wearable device (or another vital sign described herein), particularly in order to use said patient data in the method according to the present invention as described herein.

A further aspect of the present invention relates to a computer-readable non-transitory storage medium having stored thereon the computer program according to the present invention.

Furthermore, yet another aspect of the present invention relates to a data carrier signal carrying the computer program according to the present invention.

In the following, further features and advantages of the present invention as well as embodiments of the present invention shall be described with reference to the Figures, wherein

Fig. 1A    displays an overview of all components according to an embodiment of the invention and the relationships between all components. (1)-(4) are the four components of an embodiment of the method / algorithm according to the present invention. (A)-(G) are the additional components that interact with components (1)-(4),

Fig. 1B    schematically illustrates the steps of an embodiment of the method according to the present invention,

Fig. 2    schematically illustrates an iterative procedure over the number of visits carried out by an embodiment of the method / algorithm according to the present invention, wherein x denotes the current visit,

Fig. 3    shows a schematic overview of the four thyroid disease situations as an example of an application of the present invention. Normal range of thyroid hormones (free thyroxine (FT4)) is marked by the horizontal shaded area S1 and the desired time interval for normalization of FT4 values in all four thyroid diseases is highlighted by another shaded area. In congenital hyper- and hypothyroidism, FT4 should be at the upper normal reference range as rapidly as possible to protect brain development, ideally within 2 weeks. In acquired hyper- and hypothyroidism FT4 values should not increase or decrease too rapidly to avoid side effects of therapy, ideally the normal reference range is reached within 4-6 weeks. Desired FT4 increase or decline is depicted by the dashed line L1 in each graph. Over- or underdosing is depicted by solid lines L2 above and below the normal range (horizontal shaded area S1). Medication for congenital and acquired hypothyroidism is levothyroxine, and medication for congenital and acquired hyperthyroidism is carbimazole, methimazole or propylthiouracil. a) congenital hypothyroidism, b) congenital hyperthyroidism, the solid line L3 depicts the most frequent delayed peak of hyperthyroidism between day 4 and 10 of life, c) acquired hypothyroidism, d) acquired hyperthyroidism. Interrupted time axis depicts initial correction period of hyper- and hypothyroidism and long-term maintenance period of treatment,

Fig. 4    shows an example of the optimal individual dosing regimen computed by the method/computer program according to the present invention.

Fig. 5    shows the iterative procedure according to an example of the present invention with respect to acquired hyperthyroidism, and

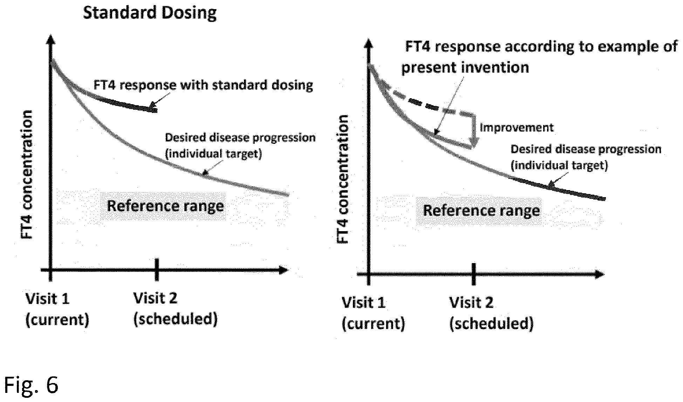

Fig. 6 illustrates the improvement (right-hand side) achieved by the present invention, here with respect to FT4 response compared to standard dosing calculation (left-hand side).

Components of an embodiment of the method/algorithm according to the present invention

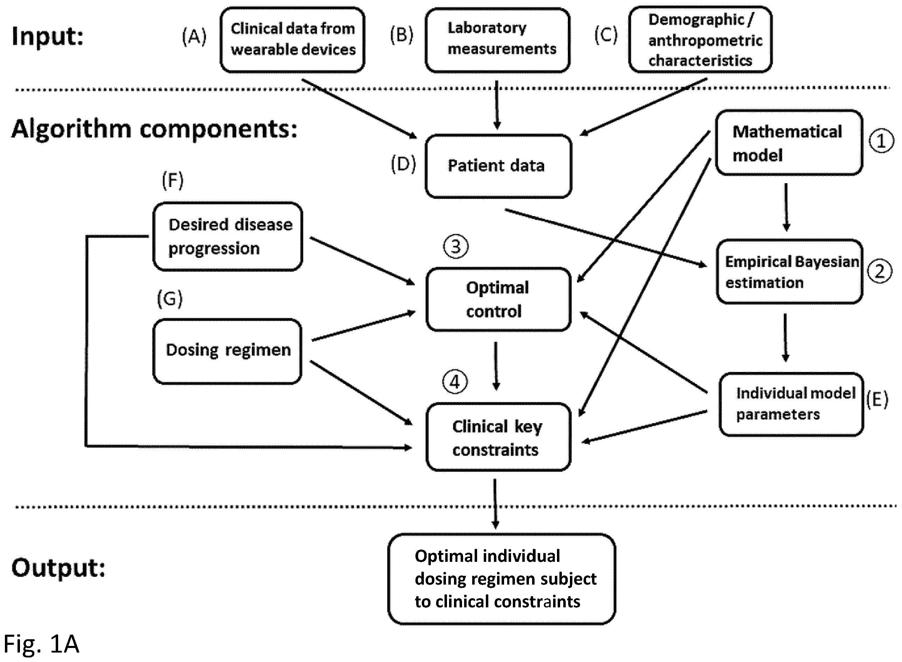

The algorithm particularly consists of four components (Figure 1A; (1)-(4)) and their interaction. In addition, all additional components which interact with the components (1)-(4) are shown in Figure 1A; (A)-(G). Explanation of all four components and their input/output relationships are presented below and in Figure 1A.

Application of a mathematical model (Figure 1A; (1))

The development of the mathematical model can be performed in two different ways. First, a mathematical model is already available. Then, this mathematical model can be applied in our algorithm and the step in this paragraph is skipped. Second, no such mathematical model is available and therefore, must be developed as follows:

Input:

1) Basic mathematical model
2) Non-linear mixed effects modeling approach
3) Retrospective patient data from a population

Output:

1) Mathematical model

Application of the empirical Bayesian estimation method (Figure 1A; (2))

The empirical Bayesian estimation method estimates the individual model parameters for a new patient based on the mathematical model and their individual patient data.

Input:

1) Mathematical model
2) Data of new patient

Output:

1) Individual model parameters of the new patient

Application of the optimal control algorithm (Figure 1A; (3))

The optimal control problem is solved in particular with the OptiDose algorithm that computes the optimal dosing regimen for an individual patient.

Input:

1) Mathematical model
2) Individual model parameters of the new patient
3) Desired disease progression provided by a mathematical function
4) Dosing regimen

Output:

1) Optimal individual dosing regimen

<u>Optionally, particularly if necessary: Adjustment of the optimal individual dosing regimen subject to clinical constraints (Figure 1A; (4))</u>

In an a-posteriori step, the optimal individual dosing regimen is adjusted to account for constraints in clinical application, e.g., limitations due to available tablet sizes.

Input:

1) Mathematical model
2) Individual model parameters of the new patient
3) Desired disease progression provided by a mathematical function
4) Clinical constraints
5) Optimal individual dosing regimen

Output:

1) Optimal individual dosing regimen subject to constraints in clinical application

Particularly, the present invention combines a mathematical model, the empirical Bayesian estimation method, an optimal control algorithm and optionally the inclusion of constraints in clinical application in order to calculate an optimal individual dosing regimen for a patient subject to said constraints.

The method / algorithm according to the present invention is an iterative procedure over the number of visits (Figure 2). At every visit, the optimal individual dosing regimen, optionally subject to clinical constraints, is computed by performing one iteration of the algorithm. This optimal individual dosing regimen is valid until the next scheduled visit.

Performing one iteration of the algorithm at a visit:

Step 1) "Estimation of the individual model parameters": Based on the available patient data at the current and the past visits, and the administered doses until the current visit (Figure 1A; (A)-(D)) the individual model parameters $\phi_i$ of the patient (Figure 1A; (E)) are estimated with the empirical Bayesian estimation method utilizing the mathematical model (Figure 1A; (1)-(2)).

Step 2) "Optimal individual dosing regimen": Estimated individual model parameters (Figure 1A; (E)) are applied to compute the optimal individual dosing regimen (Figure 1A; (1), (3), (F), (G)) for the time period until the next scheduled visit.

Step 3) (optional) "Optimal individual dosing regimen subject to clinical constraints ": The optimal individual dosing regimen (Figure 1A; (3)) is adjusted to account for constraints in clinical application (Figure 1A; (4), (E), (F), (G)).

These steps are iteratively repeated at every visit. At the first visit in this iterative process, only information at this visit is applied.

In a preferred embodiment of the present invention, the individual aspects of the present invention (particularly method, system, and computer-program according to the present invention) are applied to thyroid diseases in children and adults.

As already indicated above, thyroid hormones are essential for normal brain development, growth and puberty. However, treatment of the rare thyroid diseases congenital hypothyroidism (no or too low thyroid hormones production) and pediatric-onset Graves' disease (too high thyroid hormone production) is difficult. First, a carefully selected starting dose based on clinical experience is necessary. Second, after reaching a physiological balance of thyroid hormones, continuous adjustment of the individual dose depending on age, weight and disease severity is required to maintain euthyroidism. To mitigate the risk of negative neurological outcome, it is important to establish an optimal, individual dosing strategy that is continuously fine-tuned to account for specific needs in neonates, infants and children with hyper- or hypothyroidism.

Particularly, in an embodiment of the invention, the disease for which an optimal dosing regimen is to be determined, is one of the following four thyroid diseases which delivers a solution to the current medical problems formulated in the introduction.

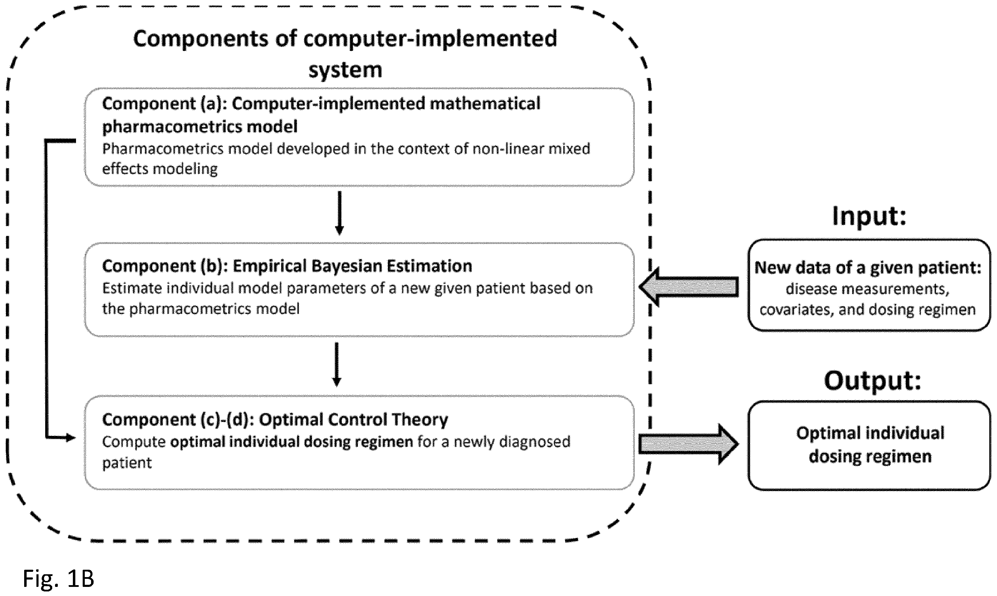

According to an embodiment, as described above, an artificial neural network (ANN) can be included in the approach in order to calculate the optimal individual dosing regimen within a short amount of time, particularly within seconds. In other words, ANNs can be used to speed up the method, system and/or computer program/algorithms according to the present invention, but are however not necessary for the invention (cf. e.g. Fig. 1; (1),(3),(F),(G) and particularly step c) of the method according to the present invention).

As an example, consider a given disease characterized by a given mathematical model. Then a sufficient number of patients, where each patient is represented by their individual model parameters, can be sampled from the given mathematical model to characterize the entire patient population with said disease. For every individual patient of this population, the optimal doses can be computed. Then an ANN is trained with the individual model parameters as input and the optimal doses as output. Hence, the trained ANN computes for given individual model parameters the optimal doses. This trained ANN can substitute step 2) "Optimal individual dosing regimen" (Figure 1A; (1),(3),(F),(G)) and claim 1 (c), respectively.

As an example, consider as basic mathematical model an indirect-response model

$$\frac{d}{dt}R(t) = k_{in} - k_{out}\left(1 + \frac{E_{max} \cdot C(d)}{EC_{50} + C(d)}\right)R(t), \quad R(0) = R^0 = \frac{k_{in}}{k_{out}}$$

in the parameterization $\theta = (R^0, k_{in}, E_{max}, EC_{50})$ where $d$ is the dose. This basic mathematical model is applied in the non-linear mixed effect modeling approach based on retrospective patient data from a population. Assuming that all model parameters in the parameterization $\theta$ follow a log-normal distribution we obtain the population parameters $\rho$.

A sufficient number $n_{suf}$ of individual patients is sampled from the population parameters $\rho$, covering a more-dimensional space with all kinds of individual parameter combinations. As example, consider the following table:

| Pat. ID | Input ANN | | | | Output ANN | | | | | |
|---------|-----------|-----------|-----------|------------|------|------|------|------|------|------|
| | $R^0$ | $k_{in}$ | $E_{max}$ | $EC_{50}$ | D1 | D2 | D3 | D4 | D5 | D6 |
| 1 | 100 | 0.1 | 5 | 0.25 | | | | | | |
| ... | ... | ... | ... | ... | | | | | | |
| $n_{suf}$ | 90 | 0.15 | 4 | 0.2 | | | | | | |

For all these individual parameters, the optimal individual dosing regimen (Step 2 or Claim 1c) optionally accounting for at least one clinical constraint (Step 3 or Claim 1d) according to a fixed dosing schedule is computed, compare section "optimal control problem". In this example, 6 doses $d = (D1, ... , D6)$ for the subsequent 6 time units are computed, and the following data set is obtained, compare the following table:

| Pat. ID | Input ANN | | | | Output ANN | | | | | |
|---------|-----------|-----------|-----------|------------|------|------|------|------|------|------|
| | $R^0$ | $k_{in}$ | $E_{max}$ | $EC_{50}$ | D1 | D2 | D3 | D4 | D5 | D6 |
| 1 | 100 | 0.1 | 5 | 0.25 | 100 | 50 | 20 | 10 | 12 | 8 |
| ... | ... | ... | ... | ... | ... | ... | ... | ... | ... | ... |
| $n_{suf}$ | 90 | 0.15 | 4 | 0.2 | 120 | 40 | 25 | 15 | 18 | 16 |

This constructed dataset is divided into a training, validation and test dataset to develop an ANN. The input of the ANN are the individual model parameters $(R^0, k_{in}, E_{max}, EC_{50})$, and the output of the ANN are the optimal doses $d = (D1, ..., D6)$ from the optimal individual dosing regimen. Hence, this trained ANN can substitute step 2) "Optimal individual dosing regimen" (Figure 1A; (1),(3),(F),(G)) and claim 1 (c), respectively.

For a new patient with new individual measurements, the empirical Bayesian estimation is applied to compute the new estimated individual model parameters ($\phi_i$ which serve as input for the ANN. The ANN is then applied to compute the optimal individual dosing regimen. Hence, step 2) "Optimal individual dosing regimen" (Figure 1A; (1),(3),(F),(G)) and claim 1 (c), respectively, is substituted by an ANN.

Patients with congenital hypothyroidism: For congenital hypothyroidism, an artificially manufactured T4 hormone (which is manufactured by different pharmaceutical companies in different galenic forms (e.g., tablets, drops), e.g., levothyroxine) is administered as substitution therapy to normalize the thyroid hormones (T3, FT3, T4, FT4, TSH) to the normal reference range. The overall concept from the main claim is applied to this disease area for pediatric patients starting at birth. A mathematical model for congenital hypothyroidism with levothyroxine substitution therapy was published by us in 2021 [Koch 2021].

Patients with acquired hypothyroidism: In analogy to congenital hypothyroidism, acquired hypothyroidism in children, pregnant women and adults can be modeled utilizing the same approaches.

Patients with acquired hyperthyroidism: For acquired hyperthyroidism, in pediatric and adult patients, two different treatment strategies are applied in clinical practice to normalize the thyroid hormones (T3, FT3, T4, FT4, TSH) to the normal reference range:

> 1) Administration of an anti-thyroid agent (e.g., carbimazole) alone to inhibit thyroid hormone overproduction
> 2) Simultaneous administration of an anti-thyroid agent (e.g., carbimazole) to inhibit thyroid function, and levothyroxine as substitution therapy to increase T4 hormones.

So far, dosing of carbimazole with or without simultaneous levothyroxine is based on laboratory testing of the thyroid hormones T3, T4, FT3, FT4, and TSH. A new approach will be the use of heart rate of the patient, a well-established very sensitive clinical sign for too high levels of thyroid hormones. Patients at diagnosis of hyperthyroidism have always too high heart rate values, which only normalize over weeks, when the thyroid hormone levels return to the normal range under carbimazole treatment. Heart rate will be monitored utilizing a wearable device. This allows to establish a relationship between FT4 and heart rate with the goal to provide medical advice and drug dosing at distance (telemedicine) based on resting heart rate without the need of clinical consultations and blood testing. This approach will be of importance especially at long distances and in low- and middle-income countries where laboratory testing is not everywhere available.

Patients with congenital hyperthyroidism: In analogy to acquired hyperthyroidism, congenital hyperthyroidism in pediatrics can be modeled by the same approaches.

Example of a mathematical model for congenital hypothyroidism

A mathematical model for levothyroxine treatment and the resulting FT4 concentration is presented [Koch 2021]. Due to the substitution therapy, this model can be considered as a pure PK model. A one-compartment model with absorption compartment and an additional endogenous production term was developed:

$$\frac{d}{dt}A_b^{T4}(t) = In^{T4}\left(t_k^{T4}, d_k^{T4}, F^{T4}\right) - k_a^{T4} \cdot A_b^{T4}(t) \qquad A_b^{T4}(0) = 0$$

$$\frac{d}{dt}T4(t) = k_a^{T4} \cdot A_b^{T4}(t) + k_{endo}^{T4} - k_{el}^{T4} \cdot T4(t) \qquad T4(0) = \frac{k_{endo}^{T4}}{k_{el}^{T4}}$$

$$C^{FT4}(t) = \frac{0.3 \cdot T4(t)}{V^{T4}(W)} \quad \text{with} \quad V^{T4}(W) = f_V^{T4} \cdot \left(\frac{W(t)}{W_{Ref}}\right)^{\beta_W}$$

where $C^{FT4}$ is the FT4 concentration resulting from levothyroxine treatment, $W$ the body weight (time-varying covariate) and $W_{Ref}$ a reference body weight (usually the average of body weight in the underlying patient population). The input function describing the dose administration is $In^{T4}$ where $t_k^{T4}$ is the k-th dosing time point, $d_k^{T4}$ the k-th dose of levothyroxine and $F^{T4}$ a multiplicative factor, e.g., describing bioavailability of levothyroxine. The absorption compartment $A_b^{T4}$ characterizes, e.g., an oral administration, and compartment $T4$ characterizes the hormone thyroxine. The absorption rate is $k_a^{T4}$, the endogenous production rate is $k_{endo}^{T4}$ and the elimination rate is $k_{el}^{T4}$. Parameter $f_V^{T4}$ is a factor relating body weight with volume of distribution $V^{T4}$ and $\beta_W$ is a shape parameter. The vector combining all model parameters reads

$$\theta = \left(k_a^{T4}, k_{endo}^{T4}, k_{el}^{T4}, f_V^{T4}, \beta_W, F^{T4}\right)$$

**Estimated** individual model parameters $\psi_i = (k_{endo,i}^{T4}, f_{V,i}^{T4}, \beta_{W,i})$ were log-normally distributed.

Example of a mathematical model for acquired hyperthyroidism

A mathematical model for carbimazole treatment, additional block-and-replace (combination with levothyroxine) therapy, and the resulting free-thyroxine (FT4) concentration is presented. Structurally, this can be considered as a PKPD model where the PK is the methimazole (metabolized carbimazole) concentration caused from the carbimazole treatment and the PD is the FT4 biomarker. The developed model can be applied to both, carbimazole mono-therapy and block-and-replace therapy.

Carbimazole is a pro-drug and several properties of the PK, such as distribution, metabolism etc., are known. A detailed carbimazole PK model is presented. Let $d_l^C$ be the dose of carbimazole at time point $t_l^C$. Carbimazole absorption is characterized by

$$\frac{d}{dt} A_b^C(t) = In^C\big(t_l^C, d_l^C\big) - k_a^C \cdot A_b^C(t) \qquad\qquad A_b^C(0) = 0$$

where $k_a^C$ is the absorption rate and $In^C$ is the dosing input function of carbimazole. Amount of carbimazole $A^C$ in the central compartment is described by

$$\frac{d}{dt} A^C(t) = k_a^C \cdot A_b^C(t) - k_t \cdot A^C(t) \qquad\qquad A^C(0) = 0$$

where $k_t$ is the metabolism transit rate. Amount of methimazole $A^M$ in the central compartment is given by

$$\frac{d}{dt} A^M(t) = f^M \cdot k_t \cdot A^C(t) - k_{el}^M \cdot A^M(t) - k_{12} \cdot A^M(t) + k_{21} \cdot P(t) \qquad A^M(0) = 0$$

$$\frac{d}{dt} P(t) = k_{12} \cdot A^M(t) - k_{21} \cdot P(t) \qquad\qquad P(0) = 0$$

where $f^M$ is the metabolism conversion factor, $k_{el}^M$ is the elimination rate, and $k_{12}$ as well as $k_{21}$ are the distribution rates for the peripheral compartment P. Concentration of methimazole is obtained by

$$C^M(t) = \frac{A^M(t)}{V^M(W)} \quad \text{with} \quad V^M(W) = f_V^M \cdot W(t)$$

where $W(t)$ is the body weight over time and $f_V^M$ is a multiplicative factor linearly relating body weight with volume of distribution of methimazole $V^M$.

The inhibitory carbimazole treatment effect on the endogenous T4 production rate via the methimazole concentration $C^M(t)$ is included utilizing an inhibitory effect term

$$\frac{d}{dt} A_b^{T4}(t) = In^{T4}\big(t_k^{T4}, d_k^{T4}, F^{T4}\big) - k_a^{T4} \cdot A_b^{T4}(t) \qquad\qquad A_b^{T4}(0) = 0$$

$$\frac{d}{dt} T4(t) \qquad\qquad\qquad\qquad T4(0) = \frac{k_{endo}^{T4}}{k_{el}^{T4}}$$

$$= k_a^{T4} \cdot A_b^{T4}(t) + k_{endo}^{T4} \cdot \left(1 - \frac{I_{max} \cdot C^M(t)}{IC_{50} + C^M(t)}\right) - k_{el}^{T4} \cdot T4(t)$$

$$C^{FT4}(t) = \frac{0.3 \cdot T4(t)}{V^{T4}(W)} \quad \text{with} \quad V^{T4}(W) = f_V^{T4} \cdot \left(\frac{W(t)}{W_{Ref}}\right)^{\beta_W}$$

where $I_{max} \leq 1$ is the maximal inhibitory drug effect and $IC_{50}$ is the methimazole concentration causing the half-maximal inhibitory effect. In addition, $In^{T4}$ is the dosing input function, $d_k^{T4}$ the dose of levothyroxine at time point $t_k^{T4}$, $F^{T4} \leq 1$ the bioavailability of levothyroxine and $k_a^{T4}$ the respective absorption rate. Parameter $f_V^{T4}$ is a factor relating body weight with volume of distribution $V^{T4}$ and $\beta_W$ is a shape parameter. $C^{FT4}$ is the FT4 concentration resulting from carbimazole mono-therapy ($In^{T4} = 0$) or block-and-replace therapy. The vector combining all model parameters reads

$$\theta = \left(k_a^C, k_t, k_{el}^M, k_{12}, k_{21}, f_V^M, k_a^{T4}, k_{endo}^{T4}, I_{max}, IC_{50}, k_{el}^{T4}, f_V^{T4}, \beta_W, F^{T4}\right)$$

Estimated individual model parameters $\psi_i = (k_{endo,i}^{T4}, IC_{50,i}, f_{V,i}^{T4}, \beta_{W,i})$ were log-normally distributed. The covariates age at baseline (AGE: time-invariant continuous) and disease severity at baseline (categorical) impact the individual endogenous T4 production rate $k_{endo,i}^{T4}$:

$$\log\left(k_{endo,i}^{T4}\right) = \log\left(k_{endo,pop}^{T4}\right) + \beta_{k_{endo}^{T4}}^{AGE} \cdot AGE + \begin{cases} 0 & \text{for severe disease} \\ \beta_{k_{endo}^{T4}}^{Cat\ 2} & \text{for moderate disease} \\ \beta_{k_{endo}^{T4}}^{Cat\ 3} & \text{for mild disease} \end{cases}$$

The continuous and time-dependent weight $W(t)$ can be modeled by, e.g., a weight progression model given by a mathematical function, e.g., the Leffler function for neonates and infants, depending on additional model parameters which can be estimated utilizing weight measurements, or as a time-varying covariate with an interpolation between weight measurements.

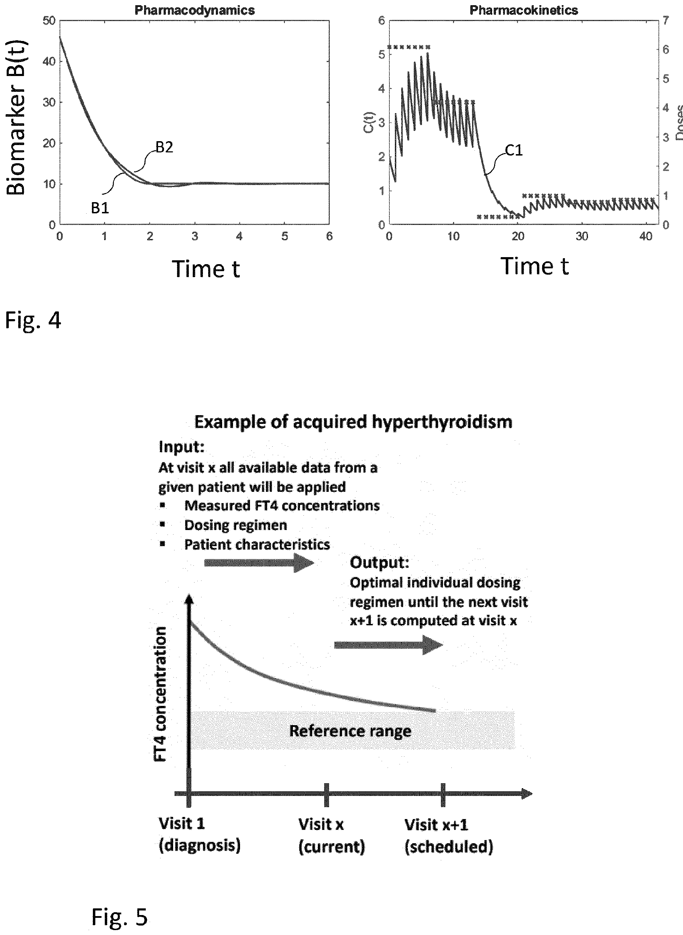

Finally, Fig. 4 depicts a schematic example of the optimal individual dosing regimen computed by the method/computer program according to the present invention.

Desired disease progression (left panel, solid curve B1) and the resulting biomarker concentration (left panel, solid curve B2) is shown for the computed optimal individual dosing regimen (right panel, crosses). The solid line C1 is the corresponding drug concentration resulting from the optimal individual dosing regimen.

## References

[Polak 2017]: Polak M, Refetoff, S, Szinnai G, van Vliet G. Part III: Thyroid Gland Disorders. Pediatric Endocrinology and Inborn Errors of Metabolism. Editors: K.

Sarafoglou, G.F. Hoffmann, K.S. Roth, McGraw-Hill Education, New York, 2nd Edition, 2017, pp. 481-512

[Leger 2014]: Léger J, Kaguelidou F, Alberti C, Carel JC. Graves' disease in children. Best Pract Res Clin Endocrinol Metab. 2014;28(2):233-243

[Belotti 2013]: Belotti P et al. (2013) Mixed-integer nonlinear optimization. Acta Numerica 22:1-131

[Lavielle 2014]: Lavielle M (2014) Mixed effects models for the population approach: models, tasks, methods and tools. Chapman & Hall / CRC Biostatistics Series Book 66

[Bassett 2018]: Bassett R, Deride J (2019) Maximum a posteriori estimators as a limit of Bayes estimators. Mathematical Programming 174:129-144

[Bachmann 2021]: Bachmann F, Koch G, Pfister M, Szinnai G, Schropp J (2021) OptiDose: computing the individualized optimal drug dosing regimen using optimal control. Journal of Optimization Theory and Applications 189:46-65

[Koch 2021]: Koch G et al. (2021) Modeling of levothyroxine in newborns and infants with congenital hypothyroidism: challenges and opportunities of a rare disease multi-center study. J Pharmacokinet Pharmacodyn 48(5):711-723

**Claims**

1. A computer-implemented method for determining an optimal individual dosing regimen of at least one drug for a patient suffering from a known disease associated with a disease progression model in accordance with the Non-Linear Mixed Effects modeling approach referred to as NLME modeling approach, wherein the method comprises the steps of:

   (a) Accessing a mathematical model adapted to model a disease progression of said disease, the mathematical model comprising a basic mathematical model together with the application of the NLME modeling approach to describe a given population of patients by its population parameters, covariates and parameter distributions, wherein the basic mathematical model is a pharmacokinetic model or a pharmacokinetic-pharmacodynamic model **characterized by** one or more differential equations, comprising a disease state as a variable characterizing the disease progression,
   (b) Using empirical Bayesian estimation to compute, based on patient data and the mathematical model, estimated individual model parameters for the patient belonging to the given population by maximizing the conditional probability density function for an individual with said population parameters and the patient data of said patient,
   (c) Calculating an optimal individual dosing regimen for the patient by solving an optimal control problem with an optimal control algorithm by minimizing a cost functional, which characterizes the difference between the disease state and a desired disease progression based on the mathematical model, and the individual model parameters of the patient, and starting with an initial control corresponding to an initial guess for the dosing regimen.

2. The method of claim 1, further comprising:
   (d) Adjusting the optimal individual dosing regimen between two available dosing sizes to account for at least one clinical constraint to yield the optimal individual dosing regimen subject to said at least one clinical constraint, by rounding to the closest available dosing size, or choosing upper or lower available dose based on the lower corresponding cost functional value.

3. The method according to one of the preceding claims, wherein the desired disease progression is given in form of a mathematical function.

4. The method according to one of the preceding claims, wherein at least a part of an algorithm performing steps (b) to (c) is approximated by an artificial neural network (ANN).

5. The method according to one of the preceding claims, wherein the disease is one of: acquired hypothyroidism, particularly autoimmune and/or non-autoimmune forms; acquired hyperthyroidism, particularly autoimmune and/or non-autoimmune forms; congenital hypothyroidism, congenital hyperthyroidism.

6. The method according to one of the preceding claims, wherein the at least one drug is selected from the group comprised of: levothyroxine, carbimazole, propylthiouracil.

7. The method according to one of the preceding claims, wherein at least a portion of said patient data is measured by a wearable device worn by the patient.

8. The method according to claim 7, wherein the wearable device is one of: a watch, particularly smart watch; a mobile phone, particularly smart phone.

9. The method according to claim 7 or 8, wherein said patient data comprises the heart rate, wherein the heart rate of the patient is measured with the wearable device.

10. A computer system for determining an optimal individual dosing regimen of at least one drug for a patient suffering from a known disease associated with a disease progression model in accordance with the Non-Linear Mixed Effects modeling approach, the system comprising at least one processor configured to perform the steps of the computer-implemented method according to one of the preceding claims.

11. The system according to claim 10, wherein the system further comprises a wearable device configured to measure at least a portion of said patient data.

**12.** A computer program product for determining an optimal individual dosing regimen of at least one drug for a patient suffering from a known disease associated with a disease progression model in accordance with the Non-Linear Mixed Effects modeling approach, comprising instructions, which when carried out on the system according to claim 10 or 11, cause the system to carry out the method according to one of the claims 1 to 9.

**13.** A computer-readable non-transitory storage medium having stored thereon the computer program according to claim 12.

**14.** A data carrier signal carrying the computer program of claim 12.

**Patentansprüche**

**1.** Computerimplementiertes Verfahren zur Bestimmung eines optimalen individuellen Dosierungsschemas mindestens eines Medikaments für einen Patienten, der an einer bekannten Krankheit leidet, die einem Krankheitsverlaufsmodell in Übereinstimmung mit dem als NLME-Modellierungsansatz bezeichneten Ansatz der nichtlinearen gemischten Effekte zugeordnet ist, wobei das Verfahren die folgenden Schritte umfasst:

(a) Zugreifen auf ein mathematisches Modell, das dazu ausgelegt ist, einen Krankheitsverlauf der Krankheit zu modellieren, wobei das mathematische Modell ein mathematisches Grundmodell zusammen mit der Anwendung des NLME-Modellierungsansatzes umfasst, um eine gegebene Patientenpopulation durch ihre Populationsparameter, Kovariaten und Parameterverteilungen zu beschreiben, wobei das mathematische Grundmodell ein pharmakokinetisches Modell oder ein pharmakokinetisch-pharmakodynamisches Modell ist, das durch eine oder mehrere Differentialgleichungen gekennzeichnet ist und einen Krankheitszustand als eine den Krankheitsverlauf charakterisierende Variable umfasst,
(b) Verwenden einer empirischen Bayes'schen Schätzung, um auf Grundlage der Patientendaten und des mathematischen Modells geschätzte individuelle Modellparameter für den Patienten, der zu der gegebenen Population gehört, zu berechnen, indem die bedingte Wahrscheinlichkeitsdichtefunktion für ein Individuum mit den Populationsparametern und den Patientendaten des Patienten maximiert wird,
(c) Berechnen eines optimalen individuellen Dosierungsschemas für den Patienten durch Lösen eines optimalen Steuerungsproblems mit einem optimalen Steuerungsalgorithmus durch Minimieren eines Kostenfunktionals, das die Differenz zwischen dem Krankheitszustand und einem gewünschten Krankheitsverlauf auf Grundlage des mathematischen Modells charakterisiert, und der individuellen Modellparameter des Patienten, wobei mit einer anfänglichen Steuerung begonnen wird, die einer anfänglichen Schätzung für das Dosierungsschema entspricht.

**2.** Verfahren nach Anspruch 1, ferner umfassend:
(d) Anpassen des optimalen individuellen Dosierungsschemas zwischen zwei verfügbaren Dosierungsgrößen, um mindestens eine klinische Einschränkung zu berücksichtigen, um das optimale individuelle Dosierungsschema unter Berücksichtigung der mindestens einen klinischen Einschränkung zu erhalten, indem auf die nächstgelegene verfügbare Dosierungsgröße aufgerundet wird oder die obere oder untere verfügbare Dosis auf Grundlage des unteren entsprechenden Kostenfunktionswertes gewählt wird.

**3.** Verfahren nach einem der vorhergehenden Ansprüche, wobei der gewünschte Krankheitsverlauf in Form einer mathematischen Funktion angegeben wird.

**4.** Verfahren nach einem der vorangegangenen Ansprüche, wobei mindestens ein Teil eines Algorithmus, der die Schritte (b) bis (c) durchführt, durch ein künstliches neuronales Netz (ANN) approximiert wird.

**5.** Verfahren nach einem der vorangegangenen Ansprüche, wobei die Krankheit eine der folgenden ist: erworbene Hypothyreose, insbesondere Autoimmun- und/oder Nicht-Autoimmunformen; erworbene Hyperthyreose, insbesondere Autoimmun- und/oder Nicht-Autoimmunformen; angeborene Hypothyreose, angeborene Hyperthyreose.

**6.** Verfahren nach einem der vorhergehenden Ansprüche, wobei das mindestens eine Medikament aus der Gruppe bestehend aus Levothyroxin, Carbimazol und Propylthiouracil ausgewählt ist.

**7.** Verfahren nach einem der vorangegangenen Ansprüche, wobei mindestens ein Teil der Patientendaten durch eine tragbare Vorrichtung gemessen wird, die der Patient trägt.

**8.** Verfahren nach Anspruch 7, wobei die tragbare Vorrichtung eines von Folgendem ist: eine Uhr, insbesondere eine Smartwatch; ein Mobiltelefon, insbesondere ein Smartphone.

**9.** Verfahren nach Anspruch 7 oder 8, wobei die Patientendaten die Herzfrequenz umfassen, wobei die Herzfrequenz des Patienten mit der tragbaren Vorrichtung gemessen wird.

**10.** Computersystem zur Bestimmung eines optimalen individuellen Dosierungsschemas mindestens eines Medikaments für einen Patienten, der an einer bekannten Krankheit leidet, die einem Krankheitsverlaufsmodell in Übereinstimmung mit dem Modellierungsansatz der nichtlinearen gemischten Effekte zugeordnet ist, wobei das System mindestens einen Prozessor umfasst, der dazu konfiguriert ist, die Schritte des computerimplementierten Verfahrens nach einem der vorhergehenden Ansprüche durchzuführen.

**11.** System nach Anspruch 10, wobei das System ferner eine tragbare Vorrichtung umfasst, die dazu konfiguriert ist, mindestens einen Teil der Patientendaten zu messen.

**12.** Computerprogrammprodukt zur Bestimmung eines optimalen individuellen Dosierungsschemas mindestens eines Medikaments für einen Patienten, der an einer bekannten Krankheit leidet, die einem Krankheitsverlaufsmodell in Übereinstimmung mit dem Modellierungsansatz der nichtlinearen gemischten Effekte zugeordnet ist, umfassend Anweisungen, die bei Ausführung auf dem System nach Anspruch 10 oder 11 das System dazu veranlassen, das Verfahren nach einem der Ansprüche 1 bis 9 auszuführen.

**13.** Computerlesbares, nicht transitorisches Speichermedium, auf dem das Computerprogramm nach Anspruch 12 gespeichert ist.

**14.** Datenträgersignal, welches das Computerprogramm nach Anspruch 12 trägt.

**Revendications**

**1.** Procédé implémenté sur ordinateur pour la détermination d'un schéma posologique optimal d'au moins un médicament pour un patient souffrant d'une maladie connue associée à un modèle de progression de maladie conformément à la stratégie de modélisation non linéaire à effets mixtes désignée par stratégie de modélisation NLME, le procédé comprenant les étapes consistant à :

(a) accéder à un modèle mathématique adapté à modéliser une progression de maladie de ladite maladie, le modèle mathématique comprenant un modèle mathématique basique conjointement avec l'application de la stratégie de modélisation NLME pour décrire une population donnée de patients par ses paramètres de population, covariables et distributions de paramètres, le modèle mathématique basique étant un modèle pharmacocinétique ou un modèle pharmacocinétique-pharmacodynamique **caractérisé par** une ou plusieurs équations différentielles, comprenant un état pathologique en tant que variable caractérisant la progression de maladie,
(b) utiliser une estimation bayésienne empirique pour calculer, sur la base de données de patient et du modèle mathématique, des paramètres de modèle individuels estimés pour le patient appartenant à la population donnée en maximisant la fonction de densité de probabilité conditionnelle pour un individu avec lesdits paramètres de population et les données de patient dudit patient,
(c) calculer un schéma posologique individuel optimal pour le patient en résolvant un problème de contrôle optimal avec un algorithme de contrôle optimal en minimisant une fonction de coût, qui caractérise la différence entre l'état pathologique et une progression souhaitée de maladie sur la base du modèle mathématique, et des paramètres de modèle individuel du patient, et en partant d'un contrôle initial correspondant à une approximation initiale pour le schéma posologique.

**2.** Procédé selon la revendication 1, comprenant en outre :
(d) ajuster le schéma posologique individuel optimal entre deux tailles posologiques disponibles pour tenir compte d'au moins une contrainte clinique afin d'obtenir le schéma posologique individuel optimal soumis à ladite au moins une contrainte clinique, en arrondissant à la plus proche taille posologique disponible, ou en choisissant la dose haute ou basse disponible, sur la base de la plus faible valeur de fonction de coût correspondante.

**3.** Procédé selon l'une quelconque des revendications précédentes, dans lequel la progression souhaitée de maladie est donnée sous forme d'une fonction mathématique.

4. Procédé selon l'une quelconque des revendications précédentes, dans lequel au moins une partie des étapes (b) et (c) d'exécution d'algorithmes est approximée par un réseau neuronal artificiel (RNA).

5. Procédé selon l'une quelconque des revendications précédentes, dans lequel la maladie est une parmi l'hypothyroïdie acquise, en particulier les formes auto-immunes et/ou non auto-immunes ; l'hyperthyroïdie acquise, en particulier les formes auto-immunes et/ou non auto-immunes ; l'hypothyroïdie congénitale, l'hyperthyroïdie congénitale.

6. Procédé selon l'une quelconque des revendications précédentes, dans lequel ledit au moins un médicament est choisi dans le groupe constitué par : la lévothyroxine, le carbimazole, le propylthiouracile.

7. Procédé selon l'une quelconque des revendications précédentes, dans lequel au moins une partie desdites données de patient est mesurée par un dispositif portable porté par le patient.

8. Procédé selon la revendication 7, dans lequel le dispositif portable est un parmi : une montre, en particulier une montre intelligente ; un téléphone mobile, en particulier un téléphone intelligent.

9. Procédé selon la revendication 7 ou 8, dans lequel lesdites données de patient comprennent la fréquence cardiaque, la fréquence cardiaque du patient étant mesurée à l'aide du dispositif portable.

10. Système informatique pour la détermination d'un schéma posologique individuel optimal d'au moins un médicament pour un patient souffrant d'une maladie connue associée à un modèle de progression de maladie conformément à la stratégie de modélisation non linéaire à effets mixtes, le système comprenant au moins un processeur configuré pour effectuer les étapes du procédé implémenté sur ordinateur selon l'une quelconque des revendications précédentes.

11. Système selon la revendication 10, le système comprenant en outre un dispositif portable configuré pour mesurer au moins une partie desdites données de patient.

12. Produit programme informatique pour la détermination d'un schéma posologique individuel optimal d'au moins un médicament pour un patient souffrant d'une maladie connue associée à un modèle de progression de maladie conformément à la stratégie de modélisation non linéaire à effets mixtes, comprenant des instructions qui, lorsqu'elles sont exécutées sur le système selon la revendication 10 ou 11, amènent le système à exécuter le procédé selon l'une quelconque des revendications 1 à 9.

13. Support d'informations non transitoire, lisible par ordinateur, sur lequel est mémorisé le programme informatique selon la revendication 12.

14. Signal porteur de données, portant le programme informatique selon la revendication 12.

Fig. 1A

**Components of computer-implemented system**

> **Component (a): Computer-implemented mathematical pharmacometrics model**
> Pharmacometrics model developed in the context of non-linear mixed effects modeling

> **Component (b): Empirical Bayesian Estimation**
> Estimate individual model parameters of a new given patient based on the pharmacometrics model

> **Component (c)-(d): Optimal Control Theory**
> Compute **optimal individual dosing regimen** for a newly diagnosed patient

**Input:**

> **New data of a given patient:** disease measurements, covariates, and dosing regimen

**Output:**

> **Optimal individual dosing regimen**

Fig. 1B

**Fig. 2**

**Fig. 3**

Fig. 4

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200273578 A1

**Non-patent literature cited in the description**

- **BACHMANN F** ; **KOCH G** ; **PFISTER M** ; **SZINNAI G** ; **SCHROPP J**. OptiDose: Computing the Individualized Optimal Drug Dosing Regimen Using Optimal Control.. *Journal of Optimization Theory and Applications*, 2021, vol. 189, 46-65
- *CHEMICAL ABSTRACTS*, 51-48-9
- *CHEMICAL ABSTRACTS*, 22232-54-8
- *CHEMICAL ABSTRACTS*, 60-56-0
- *CHEMICAL ABSTRACTS*, 51-52-5
- Part III: Thyroid Gland Disorders. **POLAK M** ; **REFETOFF, S** ; **SZINNAI G** ; **VAN VLIET G**. Pediatric Endocrinology and Inborn Errors of Metabolism. McGraw-Hill Education, 2017, 481-512
- **LÉGER J** ; **KAGUELIDOU F** ; **ALBERTI C** ; **CAREL JC**. Graves' disease in children. *Best Pract Res Clin Endocrinol Metab.*, 2014, vol. 28 (2), 233-243
- **BELOTTI P et al.** Mixed-integer nonlinear optimization.. *Acta Numerica*, 2013, vol. 22, 1-131
- **LAVIELLE M**. Mixed effects models for the population approach: models, tasks, methods and tools.. Chapman & Hall, 2014, vol. 66
- **BASSETT R** ; **DERIDE J**. Maximum a posteriori estimators as a limit of Bayes estimators.. *Mathematical Programming*, 2019, vol. 174, 129-144
- **BACHMANN F** ; **KOCH G** ; **PFISTER M** ; **SZINNAI G** ; **SCHROPP J**. OptiDose: computing the individualized optimal drug dosing regimen using optimal control.. *Journal of Optimization Theory and Applications*, 2021, vol. 189, 46-65
- **KOCH G et al.** Modeling of levothyroxine in newborns and infants with congenital hypothyroidism: challenges and opportunities of a rare disease multicenter study.. *J Pharmacokinet Pharmacodyn*, 2021, vol. 48 (5), 711-723